# EUROPEAN PATENT APPLICATION

(11) **EP 0 815 850 A1**
(43) Date of publication of application: **07.01.1998**
(21) Application number: 96903240.8
(22) Date of filing: 26.02.1996
(51) Int. Cl.: A61K 7/50

(54) **BATH OIL COMPOSITION**

(30) Priority: 27.02.1995 JP 61612/95
(71) Applicant: TSUMURA & CO., Chuo-ku, Tokyo 103 (JP)
(72) Inventor: NAKANISHI, Nobuyuki, Shizuoka 426 (JP); TSUNAKAWA, Mitsuo, Shizuoka 426 (JP); SAIMO, Yasuhiko, Shizuoka 426 (JP); YANO, Shingo, Shizuoka 426 (JP)
(74) Representative: Jones, Helen Marjorie Meredith
(86) International application number: JP9600437
(87) International publication number: WO9626713

(57) **Abstract**

A bath oil composition comprises (A) an essential oil and/or a component originating in an essential oil having a specific gravity of less than 1.0 and (B) an oleaginous component compatible with the component (A). The oleaginous component (B) has a specific gravity of 1.0 or more and the oleaginous component mixture consisting of the component (A) and the component (B) has such a specific gravity as to allow the uniform dispersion of the mixture in the bathwater as a whole. The component (A) is employed in such an amount as to give the concentration thereof in the bathwater of at least 2.0 ppm. Thus, the essential oils can well adhere to the skin and exert enhanced effects thereof on the skin surface, which might enable the percutaneous absorption of the essential oils so as to establish their effects in vivo. The composition is excellent also from the viewpoints of safety for the human body and the prevention of water pollution caused by drainage.

## Description

### Background of the Invention

### [Field of the Invention]

This invention relates to a bath oil composition. More specifically, it relates to a bath oil composition which can enhance the effects of essential oils on the skin surface by dispersing an essential oil and/or a component originating in an essential oil (to be simply referred to as "essential oils" hereinafter) in bathwater and making it/them present in an oily state to increase the deposition of the essential oils on the skin, which might enable the percutaneous absorption of the essential oils so as to establish effects thereof in vivo.

### [Related Art]

Conventional bath oils include one which contains a large amount of a surfactant to emulsify essential oils in bathwater to make the essential oils stay in the bathwater, one which contains a small amount of a surfactant to disperse essential oils over the surface of bathwater, and the like.

However, when a surfactant is used to emulsify essential oils, the deposition of the essential oils on the skin decreases and the essential oils are readily washed away by water as they are made hydrophilic. Therefore, the effects on the skin surface of the essential oils such as pain killing, easing of stiff shoulders, promotion of circulation of the blood, resistance to inflammation, resistance to bacteria and the like as well as the effects in vivo thereof obtained by percutaneous absorption cannot be expected.

When the essential oils are dispersed over the surface of bathwater, they readily volatilize into air as they are volatile components. Therefore, the effects on the skin surface and effects in vivo by percutaneous absorption of the essential oils cannot be expected.

Further, use of a large amount of a surfactant is not preferred from the viewpoints of safety for the human body and the prevention of water pollution caused by drainage.

Then, the development of a bath oil composition which increases the deposition of essential oils on the skin, can enhance the effects on the skin surface of the essential oils, and might enable the percutaneous absorption of the essential oils so as to establish the effects in vivo thereof has been desired.

### Summary of the Invention

In view of this, the inventors of the present invention have conducted intensive studies and have found that the effects on the skin surface and effects in vivo by percutaneous absorption of essential oils can be enhanced by blending the essential oils having a small specific gravity with a specific oleaginous component under predetermined conditions. The present invention is predicated upon this finding.

In other words, the bath oil composition of the present invention comprises (A) an essential oil and/or a component originating in an essential oil having a specific gravity of less than 1.0 and (B) an oleaginous component compatible with the component (A), wherein the oleaginous component (B) has a specific gravity of 1.0 or more and the component (A) is used in such an amount as to give the concentration thereof in the bathwater of at least 2.0 ppm.

The bath oil composition of the present invention improves the adhesion of essential oils to the skin by using an oleaginous component having a specific gravity of 1.0 or more to enable an oleaginous component mixture comprising an essential oil to be directly dispersed in bathwater unlike the bath oil of the prior art which uses a surfactant to emulsify and disperse essential oils in bathwater. In other words, the composition is not washed away by water easily unlike the bath oil of the prior art comprising a surfactant and can enhance the effects on the skin surface and effects in vivo by percutaneous absorption of the essential oils.

Further, since the bath oil composition of the present invention does not use a surfactant substantially, it is excellent also from the viewpoints of safety for the human body and the prevention of water pollution caused by drainage.

These objects and advantages of the present invention will become clear from the following description.

### Description of the Preferred Embodiments

The term "essential oils" as in "the essential oils used in the bath oil composition of the present invention" is a general term for volatile oleaginous substances which are hardly soluble in water and obtained by separating from leaves, rootstocks, fruits, buds and resins of plants by steam distillation, pressing, extraction or the like and refining, and comprise hydrocarbons, alcohols, acids, carbonyls, phenols, lactones, esters and the like. The term "component originating in an essential oil" refers to a constituent component of an essential oil and denotes a separated and refined component obtained by extraction, rectification, crystallization or the other simple chemical treatment and a purely synthetic component obtained from a natural compound or petrochemical by an organic synthesis reaction.

Illustrative examples of the essential oil component (A) which can be used in the bath oil composition of the present invention and has a specific gravity of less than 1.0 are given below.

That is, they include anise oil, benzoin oil, ilang ilang oil, oregano oil, orange oil, camomile oil, cajuput oil, mustard seed oil, cardamom oil, clary sage oil, clove oil, coriander oil, cypress oil, savin oil, sandalwood oil, ciderwood oil, citronella oil, cinnamon oil, jasmine oil, juniper oil, camphor oil, Japanese iris oil, ginger oil, juniper berry oil, sage oil, savory oil, geranium oil, thyme oil, turpentine oil, tarragon oil, clove oil, spirit of turpentine, nutmeg oil, olibanum oil, garlic oil, neroli oil, pine oil, basil oil, parley oil, peppermint oil, roseoil, hyssop oil, fennel oil, dwarf pine needle oil, black pepper oil, pennyroyal oil, chenopodium oil, peppermint oil, bergamot oil, rue oil, myrrh oil, majoran oil, melissa oil, eucalyptus oil, lavender oil, lemon oil, lemon glass oil, rosewood oil, rosemary oil, carvacrol, 1-carvone, camphor, camphene, coumarin, geraniol, safrole, citral, citronellal, citronellol, jasmone, dihydrojasmone, thymol, α-terpineol, nerol, hinokitiol, piperitone, methylheptenone, menthol, linalool and the like. These essential oil components (A) may be used alone or in combination of two or more.

Any oleaginous component can be used as the oleaginous component (B) which has a specific gravity of 1.0 or more and is usable in the bath oil composition of the present invention if it has high safety for the human body and is insoluble or hardly soluble in water.

Illustrative examples of the component include benzyl benzoate, isoeugenol, eugenol, acetyl trioctyl citrate, acetyl tributyl citrate, tributyl citrate, cinnamic aldehyde, ethyl cinnamate, methyl cinnamate, cinnamyl acetate, amyl salicylate, octyl salicylate, diproyplene glycol salicylate, methyl salicylate, homomenthyl salicylate, benzyl salicylate, dicarboxyethyl pantothenate, cinoxate, saccharose fatty acid ester, thianthol, paramethyl acetophenone, phenylethyl alcohol, ethyl phenylacetate, phenoxyethanol, diethyl phthalate, dimethyl phthalate, butylbenzyl phthalae, benzyl acetate, benzyl alcohol, polypropylene glycol, dimethyl maleate, ethyl methyl -phenylglycidate, methylphenyl polysiloxane, methyl polysiloxane and the like. These oleaginous components (B) having a specific gravity of 1.0 or more can be used alone or in combination of two or more.

The bath oil composition of the present invention is injected into bathwater at the time of bathing. The oleaginous component (B) having a specific gravity of 1.0 or more is selected according to the types of essential oils and has high compatibility with the essential oil component (A), and the blending ratio thereof is advantageously set such that the specific gravity of the oleaginous component mixture becomes 1.0.

Although the above blending ratio differs according to the types and combination of the oleaginous components (A) and (B), the weight ratio of the oleaginous component (B) having a specific gravity of 1.0 or more to the essential component (A) is preferably 0.1 to 10.0, particularly preferably 0.2 to 5.0.

Although the blending ratio of the oleaginous component mixture consisting of the essential oil component (A) and the oleaginous component (B) having a specific gravity of 1.0 or more to the total amount of the bath oil composition differs according to the type of the essential oil component (A), the concentration of the oleaginous component mixture in the bathwater is preferably 100 ppm or less, particularly preferably 80 ppm or less, from the viewpoint of a sticky feeling.

Further, the essential oils are contained in the bath oil composition of the present invention in such an amount as to give the concentration thereof in the bathwater of at least 2 ppm from the viewpoints of effect on skin surface and effects in vivo by percutaneous absorption thereof. If the concentration is less than 2 ppm, the effects expected from the essential oils cannot be obtained fully.

Therefore, when the bath oil composition of the present invention is a solution which is used in an amount of 20 g for 200 liters of the bathwater, the essential oils are contained in the solution in an amount of 2 wt % or more.

The bath oil composition of the present invention may further contain inorganic salts, organic acids, vitamins, proteolytic enzymes, crude drugs and extracts thereof, and other components as required in addition to the above essential components. However, the bath oil composition of the present invention does not contain a surfactant. If it contains, it is used in such an extremely limited range as not to emulsify the essential oils.

The form of the bath oil composition of the present invention is not particularly limited. It may be liquid, powdery, granular, tablet or the like if it contains the essential oil component (A) and the oleaginous component (B) having a specific gravity of 1.0 or more and the same effects can be obtained.

The following examples are given to further illustrate the present invention. However, it is to be understood that the present invention is not limited by these examples.

Bath oil compositions were produced in accordance with formulations shown in Table 1 below.

**Table 1**

| Bath oil composition | | | | | | |
|---|---|---|---|---|---|---|
| Components (wt %) | | | Example | | Comparative Example | |
| | | | 1 | 2 | 1 | 2 |
| Oleaginous component having specific gravity of 1 or more | | Methyl salicylate | 0.5 | - | - | - |
| | | Diethyl phthalate | - | 2.0 | - | - |
| | | Benzyl benzoate | 0.5 | 2.0 | - | - |
| | | Saccharose fatty acid ester | - | 1.0 | - | - |
| Essential oils | Essential oil | Camomile | 0.1 | - | 0.1 | - |
| | | Anise oil | - | 0.1 | - | 0.1 |
| | | Turpentine oil | - | 0.5 | - | 0.5 |
| | | Eucalyptus oil | - | 0.5 | - | 0.5 |
| | Component originating in essential oil | Camphor | 1.5 | 0.1 | 1.5 | 0.1 |
| | | Citronellal | 0.1 | 0.5 | 0.1 | 0.5 |
| | | Menthol | 2.5 | - | 2.5 | - |
| | | Thymol | - | 0.1 | - | 0.1 |
| Surfactant | | POE lauryl ether | - | - | 10.0 | 10.0 |
| | | coconut oil fatty acid POE glyceryl | - | - | 15.0 | 15.0 |
| Others | | Ethanol | 50.0 | 50.0 | - | - |
| | | Dipropylene glycol | 5.0 | 5.0 | - | - |
| | | 1,3 - Butylene glycol | 5.0 | 5.0 | - | - |
| | | Concentrated glycerine | 10.0 | 10.0 | - | - |
| | | Liquid paraffin | - | - | balance | balance |
| | | Isopropyl myristate | - | - | 20.0 | 20.0 |
| | | Purified water | balance | balance | - | - |
| | | Pigment | trace amount | trace amount | trace amount | trace amount |
| | | Perfume | trace amount | trace amount | trace amount | trace amount |

Thereafter, the deposition of the bath oil composition on the skin was measured in accordance with the following method. An emulsion type bath oil shown in the above Table 1 was used in Comparative Example.
(1) 10 subjects had their front arms partially bathed in the bathwater containing the bath oil of Example 1 and the bathwater containing the bath oil of Comparative Example 1 at 41 °C for 5 minutes. The residual ratio of menthol in the residual bathwater after bathing was measured to calculate the reduction rate of menthol. The calculated value was provisionally taken as the deposition on the skin. The results are shown in Table 2 below. A refreshing feeling was evaluated as well. The results are shown in Table 3 below.
Partial bathing was carried out at a concentration of 0.1 W/V % and the residual rate of menthol was measured by liquid chromatography (differential refraction detector). 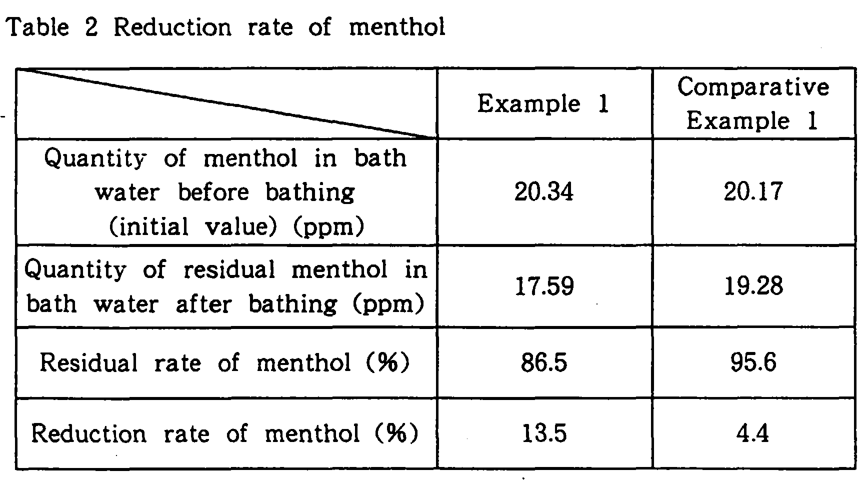 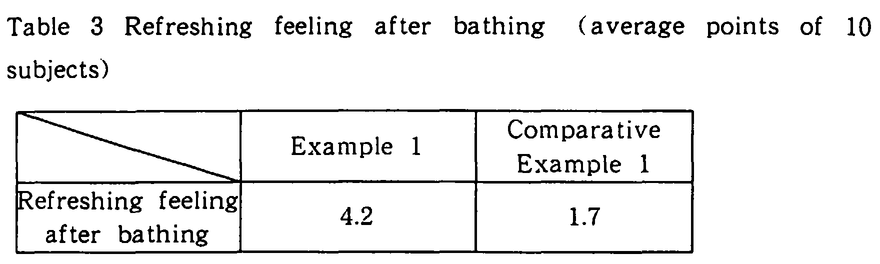 5 - Level evaluation
- 5 :: feel refreshed very much
- 4 :: feel refreshed much
- 3 :: feel refreshed
- 2 :: do not feel refreshed much
- 1 :: do not feel refreshed at all

(2) 15 subjects had their whole bodies bathed in the bath water containing 30 g (per 200 l) of the bath oil of Example 2 and the bathwater containing the same amount of the bath oil of Comparative Example 2 at 41 °C for 5 minutes and a moist feeling was evaluated. The results are shown in Table 4 below.

**Table 4**

| Example 2 | | cannot say which | Comparative Example 2 | |
|---|---|---|---|---|
| more moist | slightly more moist | | slightly more moist | more moist |
| 5 | 8 | 1 | 1 | 0 |

It has been confirmed from the results of Tables 2 to 4 that the bath oil composition of the present invention enhances the effects of essential oils on the skin surface, which might enable the percutaneous absorption of the essential oils so as to establish their effects in vivo.

### Industrial Feasibility

The bath oil composition of the present invention is characterized in that the essential oils can well adhere to the skin and exert enhanced effects thereof on the skin surface, which might enable the percutaneous absorption of the essential oils so as to establish their effects in vivo. The composition is excellent also from the viewpoints of safety for the human body and the prevention of water pollution caused by drainage.

## Claims

1. A bath oil composition comprising (A) an essential oil and/or a component originating in an essential oil having a specific gravity of less than 1.0, and (B) an oleaginous component compatible with the component (A), wherein
the oleaginous component (B) has a specific gravity of 1.0 or more and the component (A) is used in such an amount as to give the concentration thereof in the bathwater of at least 2.0 ppm.
